(19) Europäisches Patentamt
European Patent Office
Office européen des brevets

(11) Veröffentlichungsnummer: **0 433 811 B1**

(12) **EUROPÄISCHE PATENTSCHRIFT**

(45) Veröffentlichungstag der Patentschrift: **28.06.95**

(51) Int. Cl.[6]: **C07C 209/18**, C07C 209/00, C07C 211/48

(21) Anmeldenummer: **90123617.4**

(22) Anmeldetag: **08.12.90**

Die Akte enthält technische Angaben, die nach dem Eingang der Anmeldung eingereicht wurden und die nicht in dieser Patentschrift enthalten sind.

(54) **Verfahren zur Herstellung von N-alkylierten aromatischen Aminen.**

(30) Priorität: **21.12.89 DE 3942413**
**15.03.90 DE 4008257**

(43) Veröffentlichungstag der Anmeldung:
**26.06.91 Patentblatt 91/26**

(45) Bekanntmachung des Hinweises auf die Patenterteilung:
**28.06.95 Patentblatt 95/26**

(84) Benannte Vertragsstaaten:
**DE FR GB**

(56) Entgegenhaltungen:
**EP-A- 0 256 516**
**EP-A- 0 328 101**
**DE-A- 3 803 661**
**DE-A- 3 803 662**
**US-A- 4 613 705**

(73) Patentinhaber: **BAYER AG**
**D-51368 Leverkusen (DE)**

(72) Erfinder: **Immel, Otto, Dr.**
**Immenhofweg 26**
**W-4150 Krefeld (DE)**
Erfinder: **Waldmann, Helmut, Dr.**
**Henry-Th.-v.-Böttinger Strasse 15**
**W-5090 Leverkusen 1 (DE)**
Erfinder: **Braden, Rudolf, Dr.**
**Nothauserfeld 1**
**W-5068 Odenthal (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft ein Verfahren zur Herstellung von N-alkylierten Anilinen durch Umsetzung von Anilinen mit niederen Alkoholen oder durch Umsetzung mit am N-Atom alkylierten Anilinen (Transalkylierung) in Gegenwart von Niobsäure oder Tantalsäure bei erhöhter Temperatur.

N-alkylierte aromatische Amine, die erfindungsgemäß hergestellt werden können, sind Ausgangsstoffe zur Herstellung von Farbstoffen, Schädlingsbekämpfungsmitteln, Urethanen, Pharmaka, Pflanzenschutzmitteln usw. Ferner dienen sie als Mineralölzusätze und als Zusätze für Lacke oder andere polymere Systeme.

Es sind verschiedene Verfahren zur Herstellung von sekundären und tertiären aromatischen Aminen bekannt. Im allgemeinen werden sie durch Alkylierung von Anilinen mit Alkoholen in Gegenwart saurer Katalysatoren hergestellt. So ist bereits bekannt, zur Herstellung von N-alkylierten aromatischen Aminen aromatische Amine mit Alkanolen oder den zugehörigen Dialkylethern in der Gasphase umzusetzen. Als Katalysatoren werden beispielsweise Aluminiumoxid oder Silikate vorgeschlagen (beispielsweise Silikate, wie Tonsil in DE-PS 638 756). Die genannten Katalysatoren haben den Nachteil, daß ihre Aktivität bei der Alkylierung von aromatischen Aminen schnell nachläßt (Houben-Weyl, Methoden der org. Chemie, Bd. XI/1 (1957) S. 126).

Weiterhin ist aus DE-PS 617 990 und aus DE-OS 23 35 906 bekannt, daß man für die genannte Aufgabe Trägerstoffe verwenden kann, die Sauerstoffsäuren des Phosphors enthalten. Lebensdauer und Selektivität solcher Katalysatoren sind für technische Verfahren jedoch nicht befriedigend.

Weiterhin wurde in US 2.580.284 vorgeschlagen, Anilin und Methanol unter Verwendung eines Katalysators, der metallisches Kupfer auf $Al_2O_3$ und weitere Oxide, wie Zinkoxid, Cadmiumoxid, Eisenoxid, Chromoxid, Kalziumoxid, Magnesiumoxid oder Kaliumoxid enthält, umzusetzen. Solche Katalysatoren leiden unter der Abscheidung teerartiger Substanzen bereits nach relativ kurzer Zeit. Ein weiteres Verfahren zur Umsetzung von Anilin mit Methanol zu N-Methyl-anilin nach DE-OS 20 61 709 wird an einem Chrom-Katalysator durchgeführt, der Kupfer, Zink, Eisen, Nickel oder Molybdän sowie Barium, Magnesium oder Mangan enthalten kann. Dieses Verfahren hat den Nachteil, daß es unter einem Druck von 50-150 bar durchgeführt wird und daher für eine industrielle Anwendung zu kostenaufwendig ist; auch dieser Katalysator erreicht keine ausreichende Lebensdauer.

Gemäß DE-OS 2 120 641 werden Kupferchromit-Katalysatoren mit Barium, Mangan, Cer und anderen Elementen als Promotoren zur Herstellung von sekundären oder tertiären aromatischen Aminen aus aliphatischen Alkoholen und aromatische Nitroverbindungen eingesetzt. Dieses Verfahren führt aber nur zu unbefriedigenden Ausbeuten.

Für die Herstellung von am N-Atom dialkylierten Anilinen, besonders für die Herstellung von N,N-Diethylanilin, stehen nur wenige und unzureichende Verfahren zur Verfügung.

Im allgemeinen ist es zudem wünschenswert, am gleichen Katalysator je nach Bedarf variable Verhältnisse von N-Mono- zu N,N-Dialkyl-anilinen einstellen zu können.

In US 4 599 449 ist ein Verfahren zur Gasphasenalkylierung von aromatischen Aminen mit Alkoholen an Übergangsmetalloxiden der 8. Nebengruppe des Periodensystems der Elemente beschrieben. Die Ausbeute an Alkylanilinen ist jedoch sehr gering. Selbst bei einem 5-fachen Überschuß an Ethanol bleibt der Umsatz des Anilins mit maximal 38 % sehr niedrig und N,N-Diethylanilin wird nur in unzureichender Ausbeute erhalten. Darüber hinaus ist der Anteil an kernalkylierten Nebenprodukten bei diesem Verfahren sehr hoch.

DE-OS 2 335 906 lehrt die Alkylierung von Arylaminen mit Alkoholen an Kieselsäure-Katalysatoren, die mit 0,1 bis 20 Gew. % Phosphorsäure belegt sind. Zur Erzielung guter Selektivitäten ist jedoch ein sehr hoher Überschuß an Alkohol von bis zu 20 Mol-Äquivalenten erforderlich. Dies hat einerseits eine geringe Raum-Zeit-Ausbeute zur Folge, andererseits ist die Abtrennung und Recyclisierung des überschüssigen Alkohols mit einem hohen Destillationsaufwand verbunden. Um eine rasche Desaktivierung des Kontaktes zu vermeiden und eine lange Lebensdauer des Katalysators sicherzustellen, ist es darüber hinaus erforderlich, während der Alkylierung kontinuierlich Phosphorsäure und/oder Phosphorsäurealkylester zuzuführen; ein Teil dieser Phosphorverbindungen wird jedoch stets ausgetragen und verunreinigt das Reaktionsprodukt, wobei aufwendige Abtrennungen erforderlich werden.

Weiterhin ist es bekannt, Zeolithe als Katalysatoren für die Gasphasenalkylierung aromatischer Amine mit Alkoholen einzusetzen. In US 4 801 752 werden Zeolithe vom ZSM 5-Typ mit einem $SiO_2/Al_2O_3$-Verhältnis von 20-700 vorgeschlagen. Die flexible Steuerung der Ausbeute in Richtung des N,N-Diethylanilins ist jedoch nur beschränkt möglich. Unter den günstigsten Bedingungen wird für N,N-Diethylanilin eine maximale molare Selektivität von 10,1 % erzielt. Außerdem müssen bei diesem Prozeß Temperaturen bis über 400 °C angewandt werden. Zusätzlich entsteht ein signifikant hoher Anteil an nicht identifizierten kernalkylierten Nebenprodukten.

US 4 613 705 beschreibt die Alkylierung von aromatischen Aminen in Gegenwart eines Katalysators, der sich zu mind. 70 % aus einem Übergangsmetalloxid der Gruppe V B des Periodensystems der Elemente und bis zu 30 % aus Zinn(IV)-oxid zusammensetzt, beispielsweise $V_2O_5/SnO_2$, $Nb_2O_5/SnO_2$ und $Ta_2O_5/SnO_2$. Der Umsatz an aromatischen Amin ist jedoch gering, ebenso die Selektivität an N,N-Diethylanilin. Dagegen ist der Anteil an unerwünschten Nebenprodukten sehr hoch. Demzufolge erscheinen Metalloxide der 5. Nebengruppe des Periodensystems der Elemente als ungeeignete Katalysatoren zur N-Alkylierung von Anilinen im allgemeinen und zur Herstellung von N-Ethyl- und N,N-Diethylanilin im speziellen und insbesondere zur Herstellung von N,N-Diethylanilinen.

Aufgabe der vorliegenden Erfindung war es daher, die vorgenannten Nachteile zu überwinden und ein technisch ausführbares, wirtschaftlicheres Verfahren zur Herstellung von N-alkylierten Anilinen zu finden.

Es wurde nun gefunden, daß man am N-Atom alkylierte aromatische Amine aus aromatischen Aminen und niederen Alkoholen herstellen kann, wenn man die Reaktion in Gegenwart von Niobsäure oder Tantalsäure durchführt.

Die Erfindung betrifft bevorzugt ein Verfahren zur Herstellung von aromatischen Aminen der Formel

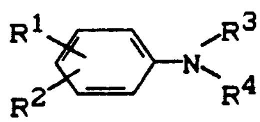

(I),

in der

$R^1$ und $R^2$ unabhängig voneinander Wasserstoff oder geradkettiges oder verzweigtes $C_1$-$C_5$-Alkyl oder $C_1$-$C_5$-Alkoxy, Fluor, Chlor, Brom oder Cyano oder einen anellierten Benzolring bedeuten können, der durch Methyl, Ethyl, Methoxy oder Ethoxy substituiert sein kann, und

$R^3$ und $R^4$ unabhängig voneinander für geradkettiges oder verzweigtes $C_1$-$C_{10}$-Alkyl oder $C_3$-$C_{10}$-Cycloalkyl stehen, wobei $R^4$ zusätzlich für Wasserstoff stehen kann,

durch Umsetzung von aromatischen Aminen der Formel

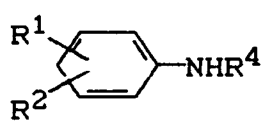

(II),

mit Alkanolen der Formel

$R^3OH$ (III),

oder den zugehörigen Ethern der Formel

$R^3$-O-$R^3$ (IV),

wobei

$R^1$, $R^2$, $R^3$ und $R^4$ die obige Bedeutung haben,

oder durch Umsetzung von aromatischen Aminen der Formel

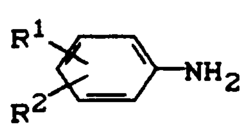

(V),

mit aromatischen Aminen der Formel

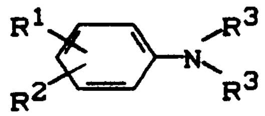

(VI),

wobei
$R^1$, $R^2$ und $R^3$ die obige Bedeutung haben,
in der Gas- oder Sumpfphase bei erhöhter Temperatur, das dadurch gekennzeichnet ist, daß man die Umsetzung in Gegenwart von Niobsäure oder Tantalsäure bei 160-400 °C, bevorzugt bei 200-330 °C durchführt.

Insbesondere bestand das Bedürfnis, ein katalytisches Verfahren zur Verfügung zu haben, mit dem eine große Anzahl verschieden substituierter Aniline in der Gasphase am N-Atom dialkyliert, besonders diethyliert werden können und das die genannten Nachteile nicht aufweist; die Alkylreste sollten insbesondere solche mit mehr als einem C-Atom sein. Die Katalysatoren sollten sich durch einfache Verfügbarkeit und hohe Standzeiten auszeichnen und hohe Umsätze bei guter Selektivität der N,N-Dialkylierung, besonders der N,N-Diethylierung gewährleisten.

Diese Forderungen im Zusammenhang mit der Dialkylierung werden erfüllt, wenn als Katalysator Niob- oder Tantalsäure (Niob(V)- oder Tantal(V)-oxid-hydrat) eingesetzt wird. Das neue Verfahren gewährleistet die geforderten hohen Ausbeuten und Umsätze, es tritt Praktisch keine Kernalkylierung auf, und der verwendete Katalysator weist Standzeiten von mehreren 1.000 h auf. Der Katalysator kann in an sich bekannter Weise durch Abbrennen mit Luft in Gegenwart von Wasserdampf regeneriert werden.

Die Erfindung betrifft daher besonders ein Verfahren zur Herstellung von N,N-Dialkylanilinen, besonders N,N-Diethylanilinen durch Umsetzung der zugrundeliegenden Aniline mit Alkanolen oder Dialkylethern, besonders Ethanol oder Diethylether, bei 200-330 °C, bevorzugt 220-310 °C, in der Gasphase bei 0,5-3 bar, das dadurch gekennzeichne ist, daß als Katalysator ein Niob(V)-oxid-hydrat $Nb_2O_5 \cdot mH_2O$ oder ein Tantal-(V)-oxid-Hydrat $Ta_2O_5 \cdot n\,H_2O$, das eine BET-Oberfläche von 5-350 $m^2/g$, bevorzugt 50-350 $m^2/g$, besonders bevorzugt 100-250, ganz besonders bevorzugt 100-200 $m^2/g$, besitzt, und Anilin und Alkanol bzw. Diethylether im Molverhältnis von 1:2-10 bzw. 1:1-5, bevorzugt 1:3-8 bzw. 1:1,5-4, besonders bevorzugt 1:3-6 bzw. 1:1,5-3, eingesetzt werden.

Es ist ein wesentliches Merkmal des erfindungsgemäßen Verfahrens, Niobsäure oder Tantalsäure als Katalysator einzusetzen. Bekanntlich ist Niobsäure ein Niobpentoxidhydrat ($Nb_2O_5 \cdot nH_2O$), das beispielsweise durch Behandlung wäßriger Lösungen von Niobsäuresalzen mit starken Mineralsäuren oder durch Behandlung von Niobsäurehalogeniden oder Niobsäureestern mit Wasser oder Basen erhalten werden kann. So gefällte Niobsäure wird getrocknet und stellt sodann eine schwerlösliche feste Verbindung dar, deren restlicher Wassergehalt nicht definiert ist, obwohl eine so hergestellte Niobsäure äußerlich ein trockenes Pulver darstellt. Die Herstellung der Niobsäure (Niobpentoxidhydrat) wird beispielsweise in Gmelins Handbuch der Anorganischen Chemie, 8. Aufl., Niob Teil B1, S. 49, beschrieben.

Tantal(V)oxid-Hydrate (Tantalsäure) für die Herstellung der erfindungsgemäß einzusetzenden Katalysatoren können durch Hydrolyse von Tantal(V)salzen, Tantal(V)-alkoholaten oder anderen geeigneten hydrolysierbaren Tantal(V)-Verbindungen hergestellt werden. Die Hydrolyse kann in bekannter Weise durch verdünnte Säuren oder verdünnte Alkalien, in vielen Fällen auch durch Wasser allein vorgenommen werden. Die Durchführung einer solchen Hydrolyse ist dem Fachmann grundsätzlich bekannt und beispielsweise im Gmelins Handbuch der Anorganischen Chemie, 8. Aufl., Tantal, Teil B1 (1970), S. 53 und T.Ushikubo, K. Wada, Chem. Lett. 1988, S. 1573 beschrieben.

Damit das Niob- oder Tantal(V)oxid-Hydrat in die für den Betrieb eines Festbett-Gasphasenreaktors günstigere stückige Form gebracht werden kann, wird beispielsweise der feuchte Niederschlag der Hydrolyse in einem Kneter durchgeknetet und in einem Granulierapparat zu Formlingen verarbeitet. Die feuchten Formkörper werden anschließend beispielsweise bei 120 °C getrocknet und 0,5 bis 5 Std. bei 200-400 °C calciniert, wobei die für das erfindungsgemäße Verfahren bevorzugte Modifikation mit der erforderlichen BET-Oberfläche entsteht.

Das hierbei entstehende Niob- bzw. Tantal(V)oxid-Hydrat weist eine BET-Oberfläche von 5-350 $m^2/g$ auf.

Zur Herstellung von Granulaten, Extrudaten oder Kugeln kann der Katalysator auch auf einen Träger aufgezogen oder mit einem Bindemittel verpreßt und granuliert werden.

Niobsäure bzw. Tantalsäure ist bezüglich des erfindungsgemäßen Verfahrens eine aktive Substanz, deren Aktivität auch durch Vermischen mit anderen Feststoffen erhalten bleibt. Geeignete Feststoffe sind beispielsweise Titandioxid, Zinkoxid, Magnesiumoxid, Eisenoxid, Siliziumdioxid, Graphit und $\alpha$-Aluminiumoxid. Gemische der Niobsäure bzw. Tantalsäure mit diesen inerten Feststoffen im Gewichtsverhältnis von 5:95 bis 99:1, bevorzugt 50:50 bis 98:2, sind beispielsweise einsetzbar. Selbstverständlich kann auch reine Niobsäure bzw. Tantalsäure eingesetzt werden. Niobsäure bzw. Tantalsäure kann beispielsweise rein oder im Gemisch mit inerten Fest stoffen als Pulver oder in granulierter Form eingesetzt werden. Die pulverige Form eignet sich hierbei eher für das Sumpfphasenverfahren, während die granulierte Form für die Anordnung als Festbett im Gasphasenverfahren geeignet ist. Die granulierte Form kann beispielsweise die Form von Tabletten, Pillen, Stäbchen oder Kugeln darstellen. Es können auch Gemische von Niobsäure und Tantalsäure eingesetzt werden. Insbesondere können solche Gemische eingesetzt werden, die dem jeweiligen Mischungsgehalt in natürlich vorkommenden Erzen entsprechen.

Niobsäure oder Tantalsäure bzw. deren Gemische mit anderen, im allgemeinen inerten Feststoffen können zur Aktivierung ferner mit starken Mineralsäuren, bevorzugt mit Fluorwasserstoffsäure oder Schwefelsäure, aktiviert werden. Eine solche Aktivierung kann beispielsweise durch Imprägnieren erfolgen. Für die Durchführung in der Sumpfphase wird Niobsäure bzw. Tantalsäure in einer Menge von 2-25 Gew.-%, bevorzugt 4-20 Gew.-%, bezogen auf das zu alkylierende aromatische Amin, eingesetzt.

Das erfindungsgemäße Verfahren wird bei einer Temperatur von 160-400 °C, bevorzugt 200-330 °C, besonders bevorzugt 220-330 °C, und bei einem Druck von 0,5-100 bar, bevorzugt 3-100 bar in der Sumpfphase bzw. von 0,5-3 bar in der Gasphase durchgeführt. Bevorzugt ist hierbei die Durchführung bei 200-330 °C in der Gasphase.

Die Katalysatorbelastung LHSV (Liquid Hourly Space Velocity) kann im Bereich von 0,1-4 l/l/h, bevorzugt von 0,3-2 l/l/h, variiert werden. Die LHSV ist hierbei als das Verhältnis des Volumens des flüssigen Anilin/Alkanol/Dialkylether-Gemisches pro Katalysatorvolumen pro Stunde vor dem Verdampfen definiert.

Als geradkettiges oder verzweigtes $C_1$-$C_5$-Alkyl oder $C_1$-$C_5$-Alkoxy in den obengenannten Formeln seien beispielsweise genannt: Methyl, Ethyl, Propyl, Isopropyl, Butyl, Isobutyl, die verschiedenen Pentylreste, Methoxy, Ethoxy, Propoxy, Isopropoxy, Butoxy, Isobutoxy, die verschiedenen Pentyloxyreste. $C_6$-$C_{10}$-Alkyl ist weiterhin beispielsweise geradkettiges oder verzweigte Hexyl, Octyl oder Decyl. In bevorzugter Weise seien als solche Reste Methyl, Ethyl, Methoxy und Ethoxy, besonders bevorzugt Methyl und Methoxy genannt. $C_3$-$C_{10}$-Cycloalkyl ist beispielsweise Cyclopropyl, Cyclobutyl, Cyclopentyl, Cyclohexyl, Cycloheptyl, Cyclooctyl, Methyl-cyclopentyl, Methyl-cyclohexyl, 4-tert.-Butyl-cyclohexyl, Menthyl, bevorzugt Cyclopropyl, Cyclopentyl und Cyclohexyl.

In bevorzugter Weise wird von aromatischen Aminen der Formel (V) ausgegangen, worin $R^1$ und $R^2$ die obige Bedeutung haben. In weiterhin bevorzugter Form wird von aromatischen Aminen der Formel

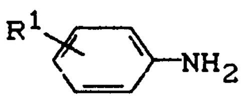 (VII),

ausgegangen, worin

$R^1$ die obige Bedeutung hat.

Eine beispielhafte, keineswegs erschöpfende Aufzählung von aromatischen Aminen für das erfindungsgemäße Verfahren ist die folgende: Anilin, 1-Naphthylamin, o-, m-, p-Toluidin, o-, m-, p-Ethylanilin, die isomeren Xylidine, wie 2,3-, 2,4-, 2,5-, 2,6-, 3,4- oder 3,5-Dimethylanilin, 4-Methyl-naphthylamin-1. Besonders wichtig ist das erfindungsgemäße Verfahren für die Alkylierung und Transalkylierung, ausgehend von Anilin und den Toluidinen.

Bei der Durchführung des erfindungsgemäßen Verfahrens in der Gasphase wird das als Ausgangsmaterial eingesetzte aromatische Amin im allgemeinen vor Eintritt in den Reaktionsraum verdampft und über den als Festbett angeordneten Katalysator geleitet. Das verdampfte Ausgangsmaterial kann mit einem Trägergasstrom verdünnt werden, beispielsweise mit $H_2$, $N_2$, $H_2O$-Dampf, $CH_4$ und anderen im erfindungsgemäßen Verfahren inerten Gasen.

Alkanole für das erfindungsgemäße Verfahren sind offenkettige Alkanole mit 1-10 C-Atomen, bevorzugt mit 1-6 C-Atomen, besonders bevorzugt mit 1-2 C-Atomen, und cyclische Alkanole mit 3-10 C-Atomen, bevorzugt mit 3, 5 oder 6 C-Atomen, wie Methanol, Ethanol, Propanol, i-Propanol, Butanol, i-Butanol, Pentanol, i-Pentanol, die isomeren Hexanole, Heptanole, Octanole oder Decanole, Cyclopropanol, Cyclobutanol, Cyclopentanol, Cyclohexanol, Cycloheptanol oder Cyclooctanol, sowie 2-, 3- oder 4-Methyl-cyclohexanol, 2-Ethyl-cyclohexanol, 3,3,5-Trimethylcyclohexanol, 4-tert.-Butyl-cyclohexanol und Menthol ($C_{10}$).

Anstelle der Alkanole $R^3OH$ (III) können auch die zugehörigen Ether $R^3$-O-$R^3$ (IV) eingesetzt werden; der Einsatz der Alkanole ist jedoch bevorzugt.

Anstelle der Alkanole können auch die korrespondierenden Olefine und Wasser eingesetzt werden, beispielsweise Ethylen und Wasser anstelle von Ethanol wobei sich möglicherweise in einem vorgelagerten Reaktionsschritt das Alkanol bildet, das mit dem aromatischen Amin erfindungsgemäß weiterreagiert.

Das Molverhältnis des Alkanols zu dem am N-Atom zu alkylierenden aromatischen Amin beträgt 0,3-20:1, bevorzugt 0,7-15:1, besonders bevorzugt 0,7-7:1. Beim Einsatz der zugehörigen Ether ist für die Berechnung des Molverhältnisses 1 Mol $R^3$-O-$R^3$ 2 Molen $R^3$-OH gleichzusetzen.

Mit solchen molaren Mengen an Alkanol können aus aromatischen Aminen der Formel (II), in denen $R^4$ die Bedeutung von Wasserstoff hat und demnach identisch ist mit den aromatischen Aminen der Formel (V) sekundäre oder tertiäre aromatische Amine der Formel (I) hergestellt werden. Desweiteren können auch aus sekundären aromatischen Aminen der Formel (II), in denen $R^4$ verschieden von Wasserstoff ist, tertiäre aromatische Amine der Formel (I) hergestellt werden. Im allgemeinen entsteht hierbei ein Gemisch aus sekundären und tertiären aromatischen Aminen, die gegebenenfalls noch primäres aromatisches Amin enthalten, falls dieses als Ausgangsmaterial eingesetzt wird. Solche Gemische können in einer dem Fachmann bekannten Weise beispielsweise destillativ aufgetrennt werden. Vielfach ist es wünschenswert, einen größeren Anteil oder ausschließlich sekundäre aromatische Amine herzustellen, die also am N-Atom nur einfach alkyliert sind. In einem solchen Fall kann etwa mitentstandenes tertiäres Amin (am N-Atom zweifach alkyliert), sofern es nicht einer eigenen Verwendung zugeführt werden kann, dem Ausgangsgemisch des erfindungsgemäßen Verfahrens zugesetzt werden. Durch diesen Zusatz kann die erneute Bildung von tertiären Aminen zurückgedrängt werden oder das tertiäre Amin überträgt in einer Transalkylierungsreaktion eine der Alkylgruppen auf am N-Atom nicht substituiertes aromatisches Amin der Formel (V). Hierbei kann die Menge des einzusetzenden Alkanols oder des zugehörigen Ethers reduziert werden. Gleichermaßen ist es möglich, ohne Mitverwendung eines Alkanols bzw. des zugehörigen Ethers nur von einem zu alkylierenden aromatischen Amin (V) und einem am N-Atom zweifach alkylierten aromatischen Amin der Formel (VI) auszugehen und nur die beschriebene Transalkylierung durchzuführen. Alle diese Möglichkeiten machen das erfindungsgemäße Verfahren sehr variabel in bezug auf die Herstellung einfach bzw. zweifach am N-Atom alkylierter Amine.

Für den Fall der überwiegend nur einfachen N-Alkylierung ist es weiterhin bevorzugt, einen noch engeren molaren Bereich von 0,8-5 Mol Alkanol pro Mol primäres aromatisches Amin einzusetzen. Dem Bestreben nach nur einfacher N-Alkylierung kommt das erfindungsgemäße Verfahren in guter Weise entgegen. Es wurde nämlich überraschend gefunden, daß auch noch bei solchen relativ hohen molaren Mengen an Alkanol, nämlich bis zu 5 Mol pro Mol des primären aromatischen Amins, fast ausschließlich am N-Atom monoalkylierte aromatische Amine entstehen, wenn man im unteren Temperaturbereich und im unteren Bereich der Verweilzeiten bleibt. Als Reaktionsbedingungen hierfür seien beispielsweise genannt: 160-220 °C und Verweilzeiten von 0,5-8 sek bzw. die reziprok daraus zu berechnende Katalysatorbelastung.

Umgekehrt führen höhere Reaktionstemperaturen, beispielsweise 200-330 °C und längere Verweilzeiten, beispielsweise 5-20 sek in Verbindung mit höheren molaren Überschüssen an Alkanol im genannten Bereich zu einer verstärkten Dialkylierung am N-Atom.

Ein besonderer Vorteil des erfindungsgemäßen Verfahrens ist es, das Verhältnis von N-Mono- zu N,N-Dialkyl-anilin in weiten Grenzen variabel zu gestalten. Dafür bevorzugt geeignete Alkanole und Dialkylether sind solche mit $C_1$-$C_3$- Resten, besonders bevorzugt Ethanol und Diethylether.

Eine weitere Variante des erfindungsgemäßen Verfahrens im Rahmen der zweifachen N-Alkylierung besteht darin, daß ein sekundäres aromatisches Amin der Formel (II), in der $R^4$ verschieden von Wasserstoff ist, mit einem Alkanol oder einem zugehörigen Ether alkyliert wird, dessen Rest $R^3$ verschieden vom Rest $R^4$ ist und somit tertiäre aromatische Amine der Formel (I) gebildet werden, die am N-Atom neben dem aromatischen Kern zwei verschiedene Alkylreste tragen. Entstehende Stoffgemische können nach bekannten Methoden getrennt werden.

<u>Beispiel 1</u>

In ein für die katalytische Gasphasenreaktion geeignetes Reaktionsrohr von ca. 17 mm Innendurchmesser wurden 11,4 g (15 ml) Niobsäure gefüllt. Diese Niobsäure war unter Zusatz von 3,5 % Graphit zu Pillen (d = 3 mm) verpreßt worden. Über die Katalysatorschicht wurde bei 250 °C ein Gasgemisch aus Anilin und Methanol im Molverhältnis 1:4 mit einer Geschwindigkeit von 0,65 g/ml•h geleitet. Das im Verlauf von 65 Stunden entstandene Reaktionsprodukt hatte lt. Gaschromatographie folgende Zusammensetzung:

| | |
|---|---|
| Anilin: | 0,1 % |
| N-Methylanilin: | 3 % |
| N,N-Dimethylanilin: | 95,1 % |
| Nebenprodukte: | 1,8 % |

Beispiel 2

20 Gew.-Teile pulverförmige Niobsäure, 80 Gew.-Teile $TiO_2$-Pulver (Anatas) und 3 Gew.-Teile Graphitpulver wurden intensiv vermischt und zu Pillen (d = 5 mm) verpreßt. 15 ml (13,8 g) des so hergestellten Katalysators wurden in ein 17 mm weites Reaktionsrohr gefüllt. Über diese Katalysatorschicht wurde bei 250 °C ein Gasgemisch aus Anilin und Ethanol im Molverhältnis 1:4 mit einer Geschwindigkeit von 0,43 g/ml•h geleitet. Das im Verlauf von 65 Stunden entstandene Reaktionsgemisch wurde kondensiert und analysiert. Es ergab sich folgende Zusammensetzung des Reaktionsgemisches:

| | |
|---|---|
| Anilin: | 9,1 % |
| N-Ethylanilin: | 46,2 % |
| N,N-Diethylanilin: | 43,3 % |
| Nebenprodukte: | 1,4 % |

Beispiel 3

Ein 1-l-Rührautoklav wurde mit 10 g pulverförmiger Niobsäure, 189,4 g Anilin und 260,6 g Methanol (Molverhältnis 1:4) beschickt. Der Autoklav wurde mit Stickstoff gespült, um die Luft zu verdrängen. Dann wurden 5 bar Stickstoff aufgedrückt und der Inhalt des Autoklaven im Verlauf von einer Stunde auf 250 °C erwärmt. Das Reaktionsgemisch zeigte, nachdem 1 bzw. 4 Stunden auf 250 °C erhitzt worden war, folgende Zusammensetzung:

| | 1 Stunde | 4 Stunden |
|---|---|---|
| Anilin: | 17,7 % | 0,7 % |
| N-Methylanilin: | 39,3 % | 10,3 % |
| N,N-Dimethylanilin: | 42,3 % | 87,2 % |
| Nebenprodukte: | 0,7 % | 1,8 % |

Beispiel 4

Ein 1-l-Rührautoklav wurde mit 20 g pulverförmiger Niobsäure, 151 g Anilin und 299 g Ethanol (Molverhältnis 1:4) beschickt. Nachdem die Luft durch Stickstoff verdrängt worden war, wurde das Gemisch 6 Stunden auf 220 °C erhitzt. Das Reaktionsgemisch entsprach folgender Zusammensetzung:

| | |
|---|---|
| Anilin: | 56,2 % |
| N-Ethylanilin: | 41,5 % |
| N,N-Diethylanilin: | 2,0 % |
| Nebenprodukte: | 0,3 % |

Beispiel 5

Zur Herstellung von N-Methylanilin wurden je 0,5 Mol Anilin (47 g) und N,N-Dimethylanilin (61 g) sowie 10 g pulverförmige Niobsäure in einem 0,25 l-Schüttelautoklaven 4 Stunden auf 250 °C erhitzt. Die Analyse des Reaktionsproduktes zeigte, daß eine beträchtliche Transalkylierung stattfand:

| | |
|---|---|
| Anilin: | 32,2 % |
| N-Methylanilin: | 24,7 % |
| N,N-Dimethylanilin: | 42,9 % |
| Nebenprodukte: | 0,2 % |

Beispiel 6

In der Apparatur wie in Beispiel 1, gefüllt mit Tantalsäure/Graphit wurde bei 220 °C ein Gasgemisch aus Anilin und Ethanol im Molverhältnis 1:4 mit einer Geschwindigkeit von 0,49 g/ml•h geleitet. Das im Verlauf von 122 h gebildete Reaktionsgemisch hatte folgende Zusammensetzung:

| | |
|---|---|
| Anilin: | 38,7 % |
| N-Ethylanilin: | 51,6 % |
| N,N-Diethylanilin: | 8,7 % |
| Nebenprodukte: | 1,0 %. |

Beispiel 7 Katalysator-Zubereitung

Eine Lösung von 100 g Tantalpentachlorid in 100 ml abs. Ethanol wurde in eine Lösung von 100 g Kaliumhydroxid in Ethanol in einem konstanten Strom eingetragen. Nach 24-stündigem Altern wurde das Präzipitat über eine Nutsche abgetrennt und solange mit siedendem Wasser gewaschen, bis das ablaufende Waschwasser frei von Chlorid-Ionen war.

Der Niederschlag wurde dann ca. 15 min in einem Kneter geknetet und anschließend in einem Granulierapparat zu Formlingen von 1-2 mm Durchmesser verarbeitet. Das feuchte Granulat wurde bei 120 °C getrocknet und 3 Std. bei 300 °C calciniert. Das so erhaltene Granulat wies eine BET-Oberfläche von 153 $m^2$/g auf.

Beispiele 8 und 9: Anilin-Ethylierung

In einem senkrecht stehenden Reaktionsrohr von 40 cm Länge und einem Durchmesser von 30 mm wurden 20 ml des Tantal(V)oxid-hydrates aus Beispiel 7, das eine mittlere Korngröße von 1-2 mm besaß, eingebracht. Mittels eines organischen Wärmeträgers ließ sich der Reaktor thermostatisieren. Die Temperatur in der Katalysatorschüttung konnte mittels eines beweglichen Thermoelements gemessen werden. Über

eine Dosiervorrichtung wurde ein Gemisch von Anilin und Ethanol in einen Verdampfer eindosiert, dort in die Gasphase übergeführt und über den Kontakt geleitet. Die Dosiergeschwindigkeit betrug 20 ml/h flüssiges Gemisch; dies entsprach einer Katalysatorbelastung (LHSV) von 1,0 l/l/h. Das Reaktionsprodukt wurde auskondensiert und gaschromatographisch analysiert. Die Gewichtszusammensetzung der Reaktionsprodukte ist in Tab. 1 zusammengestellt.

Tab. 1: N,N-Diethylierung von Anilin mit Ethanol an Tantal(V)-oxid-Hydrat

| Bsp. | Anilin Ethanol (molar) | Gew.-%-Anteile im Produkt Anilin | N-Monoethyl-anilin | N,N-Diethyl-anilin | Rest | Temperatur (°C) |
|---|---|---|---|---|---|---|
| 8 | 1:4 | 3,6 | 47,8 | 47,8 | 0,8 | 290 |
| 9 | 1:6 | 3,7 | 37,7 | 56,3 | 2,3 | 290 |

Nach 90 h Laufzeit ist das Ergebnis unverändert

Beispiel 10

Pulverförmige Niobsäure, Typ AD/620 der Firma Companhia Brasileira de Metalurgia e Mineracao wurde nach Zumischung von 3,5 % Graphit zu Tabletten verpreßt (d = 5 mm).

17,4 g (15 ml) der tablettierten Niobsäure wurden in ein 17 mm weites Reaktionsrohr gefüllt. Über diese Katalysatorschicht wurde bei 230 °C bei atmosphärischen Druck ein Gemisch aus Anilin und Ethanol im Molverhältnis 1:4 mit einer Geschwindigkeit von 0,45 g/ml*h geleitet. Das im Verlauf von 191 Stunden entstandene Reaktionsgemisch wurde kondensiert und analysiert. Es ergab sich folgende Zusammensetzung des Reaktionsproduktes.

| | |
|---|---|
| Anilin | : 1,2 % |
| N-Ethylanilin | : 24,6 % |
| N,N-Diethylanilin | : 71,3 % |
| Nebenprodukte | : 2,9 % |

Beispiel 11-15

In einem Rohrreaktor mit 1 l Niobsäure als Katalysator wurden bei einem Einsatz von 4 Mol Ethanol und 1 Mol Anilin (gemäß gaschromatographischer (GC)-Analyse 48,9 Gew.-% Anilin und 51,1 Gew.-% Ethanol, bestimmt als Flächenprozente) und bei den verschiedenen angegebenen Temperaturen und Katalysatorbelastungen folgende Werte erhalten:

Tabelle 2 (Beispiele 11-15)

| Nr. | Temp. °C | GHSV 1/l.h | Produktanalyse (GC-Flächen-%) DEE | EtOH | An. | MEA | DEA | KAA | An.-Umsatz (%) | Selektivität in Mol-% MEA | DEA | ges. |
|---|---|---|---|---|---|---|---|---|---|---|---|---|
| 11 | 270 | 0,30 | 4,6 | 6,4 | 0,3 | 19,6 | 57,8 | 9,9 | 99,5 | 26,3 | 62,9 | 89,2 |
| | 280 | 0,30 | 6,7 | 5,7 | 0,4 | 20,3 | 46,6 | 18,5 | 99,3 | 27,7 | 51,7 | 79,5 |
| | 290 | 0,30 | 6,7 | 3,8 | 1,1 | 25,7 | 34,8 | 26,2 | 98,1 | 34,2 | 37,6 | 71,7 |
| | 300 | 0,30 | 5,2 | 2,6 | 2,2 | 29,2 | 26,0 | 31,5 | 96,4 | 38,5 | 27,8 | 66,3 |
| | 310 | 0,30 | 4,3 | 1,7 | 3,6 | 31,0 | 18,9 | 39,0 | 94,3 | 39,7 | 19,7 | 59,4 |
| 12 | 270 | 0,50 | 3,1 | 8,6 | 0,9 | 23,5 | 58,1 | 4,9 | 98,5 | 31,5 | 63,2 | 94,7 |
| | 280 | 0,50 | 4,1 | 8,4 | 0,8 | 23,5 | 55,1 | 6,9 | 98,6 | 31,8 | 60,6 | 92,4 |
| | 290 | 0,50 | 4,9 | 6,3 | 0,7 | 24,4 | 50,5 | 11,9 | 98,8 | 32,5 | 54,6 | 87,1 |
| | 300 | 0,50 | 5,4 | 5,0 | 1,1 | 27,1 | 42,6 | 17,2 | 98,1 | 35,8 | 45,7 | 81,5 |
| | 310 | 0,50 | 5,1 | 3,3 | 2,0 | 30,0 | 31,8 | 26,1 | 96,7 | 39,0 | 33,5 | 72,5 |
| 13 | 270 | 0,75 | 2,7 | 10,5 | 1,3 | 24,5 | 56,1 | 4,1 | 97,7 | 33,4 | 62,1 | 95,5 |
| | 280 | 0,75 | 3,8 | 8,9 | 0,9 | 23,1 | 55,8 | 6,6 | 98,4 | 31,3 | 61,4 | 92,7 |
| | 290 | 0,75 | 4,2 | 7,7 | 1,0 | 24,6 | 50,9 | 10,5 | 98,3 | 33,0 | 55,5 | 88,5 |
| | 300 | 0,75 | 5,1 | 5,8 | 1,0 | 26,0 | 44,9 | 15,7 | 98,3 | 34,6 | 48,5 | 83,0 |
| | 310 | 0,75 | 5,0 | 4,3 | 1,6 | 29,3 | 36,8 | 21,5 | 97,4 | 38,2 | 39,0 | 77,2 |
| 14 | 270 | 1,00 | 2,3 | 17,8 | 3,2 | 26,8 | 46,7 | 2,6 | 94,1 | 40,1 | 56,7 | 96,8 |
| | 280 | 1,00 | 3,0 | 11,4 | 2,0 | 26,0 | 52,0 | 4,6 | 96,5 | 36,1 | 58,7 | 94,8 |
| | 290 | 1,00 | 4,1 | 9,0 | 1,4 | 25,0 | 51,6 | 7,4 | 97,6 | 34,3 | 57,5 | 91,8 |
| | 300 | 1,00 | 5,0 | 7,5 | 1,1 | 25,6 | 48,0 | 11,1 | 98,1 | 34,8 | 53,0 | 87,8 |
| | 310 | 1,00 | 5,6 | 5,9 | 1,5 | 27,6 | 41,6 | 16,3 | 97,5 | 37,0 | 45,3 | 82,3 |
| 15 | 270 | 1,50 | 3,2 | 39,6 | 5,4 | 27,6 | 22,2 | 1,6 | 87,0 | 58,8 | 38,4 | 97,2 |
| | 280 | 1,50 | 3,3 | 23,0 | 4,3 | 25,5 | 40,2 | 2,8 | 91,5 | 42,2 | 54,0 | 96,2 |
| | 290 | 1,50 | 4,2 | 12,2 | 2,7 | 26,2 | 48,0 | 5,4 | 95,2 | 37,7 | 56,0 | 93,7 |
| | 300 | 1,50 | 5,0 | 9,3 | 2,0 | 26,0 | 46,5 | 9,4 | 96,5 | 36,4 | 52,9 | 89,3 |
| | 310 | 1,50 | 5,3 | 7,7 | 1,9 | 27,4 | 43,0 | 12,4 | 96,7 | 37,9 | 48,2 | 86,1 |

| | |
|---|---|
| GHSV | (Gaseous Hourly Space Velocity) = Katalysatorbelastung in l/l·h |
| DEE | Diethylether |
| EtOH | Ethanol |
| An. | Anilin |
| MEA | Mono-N-ethylanilin |
| DEA | Di-N-ethylanilin |
| KAA | kernalkylierte Aniline |
| An. | Anilin |

## Patentansprüche

1. Verfahren zur Herstellung von am N-Atom alkylierten aromatischen Aminen aus aromatischen Aminen und niederen Alkoholen, dadurch gekennzeichnet, daß man die Reaktion in Gegenwart von Niobsäure oder Tantalsäure durchführt.

2. Verfahren zur Herstellung von aromatischen Aminen der Formel

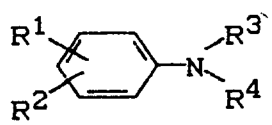

in der

$R^1$ und $R^2$ unabhängig voneinander Wasserstoff oder geradkettiges oder verzweigtes $C_1$-$C_5$-Alkyl oder $C_1$-$C_5$-Alkoxy, Fluor, Chlor, Brom oder Cyano oder einen anellierten Benzolring bedeuten können, der durch Methyl, Ethyl, Methoxy oder Ethoxy substituiert sein kann, und

$R^3$ und $R^4$ unabhängig voneinander für geradkettiges oder verzweigtes $C_1$-$C_{10}$-Alkyl oder $C_3$-$C_{10}$-Cycloalkyl stehen, wobei $R^4$ zusätzlich für Wasserstoff stehen kann,

durch Umsetzung von aromatischen Aminen der Formel

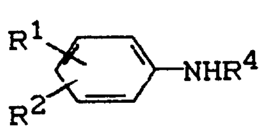,

mit Alkanolen der Formel

$R^3OH$

oder den zugehörigen Ethern der Formel

$R^3$-O-$R^3$,

wobei

$R^1$, $R^2$, $R^3$ und $R^4$ die obige Bedeutung haben,
oder durch Umsetzung von aromatischen Aminen der Formel

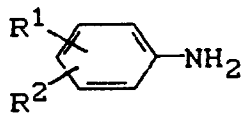
,

mit aromatischen Aminen der Formel

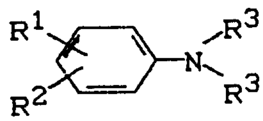
,

wobei
$R^1$, $R^2$ und $R^3$ die obige Bedeutung haben,
in der Gas- oder Sumpfphase bei erhöhter Temperatur nach Anspruch 1, dadurch gekennzeichnet, daß man die Umsetzung in Gegenwart von Niobsäure oder Tantalsäure bei 160-400 ° C, bevorzugt bei 200-330 ° C durchführt.

**3.** Verfahren nach Anspruch 1, dadurch gekennzeichnet, daß man die Niobsäure oder Tantalsäure mit einem inerten Feststoff, wie Titandioxid, Zinkoxid, Magnesiumoxid, Eisenoxid, Siliziumdioxid, Graphit oder $\alpha$-Aluminiumoxid im Mengenverhältnis von 5:95 bis 99:1 vermischt und pelletisiert.

**4.** Verfahren nach Anspruch 2, dadurch gekennzeichnet, daß man bei der Umsetzung 0,3-20 Mol Alkanol pro Mol des aromatischen Amins einsetzt.

**5.** Verfahren nach Anspruch 2, dadurch gekennzeichnet, daS bei Umsetzung in der Gasphase eine Katalysatorbelastung von 0,1-4 l/l/h, bevorzugt 0,3-2 l/l/h, zu alkylierendes aromatisches Amin je Liter Katalysator je Stunde eingestellt wird.

**6.** Verfahren nach Anspruch 2, dadurch gekennzeichnet, daß bei Umsetzung in der Sumpfphase Niobsäure oder Tantalsäure in einer Menge von 2-25 Gew.-%, bevorzugt 4-20 Gew.-%, bezogen auf das zu alkylierende aromatische Amin, eingesetzt wird.

**7.** Verfahren nach Anspruch 2, dadurch gekennzeichnet, daß in der Gasphase bevorzugt bei 200-330 ° C gearbeitet wird.

**8.** Verfahren nach Anspruch 2, dadurch gekennzeichnet, daß zur Bevorzugung der N-Monoalkylierung gegenüber der N,N-Dialkylierung bei 160-220 ° C und zur Bevorzugung der N,N-Dialkylierung gegenüber der N-Monoalkylierung bei 200-330 ° C gearbeitet wird.

**9.** Verfahren zur Herstellung von am N-Atom dialkylierten, bevorzugt diethylierten aromatischen Aminen nach Anspruch 1, dadurch gekennzeichnet, daß bei 200-330 ° C, bevorzugt bei 220-310 ° C und mit einem Molverhältnis von Anilin zu Alkanol bzw. dem entsprechenden Dialkylether von 1:2-10 bzw. 1:1-5, bevorzugt 1:3-8 bzw. 1:1,5-4, besonders bevorzugt 1:3-6 bzw. 1:1,5-3 gearbeitet wird.

**10.** Verfahren nach Anspruch 1, dadurch gekennzeichnet, daS der Katalysator eine BET-Oberfläche von 5-350 $m^2/g$, bevorzugt 50-350 $m^2/g$, besonders bevorzugt 100-250 $m^2/g$, ganz besonders bevorzugt 100-200 $m^2/g$ besitzt.

## Claims

1. Process for the preparation of aromatic amines, alkylated on the N-atom, from aromatic amines and lower alcohols, characterized in that the reaction is carried out in the presence of niobic acid or tantalic acid.

2. Process for the preparation of aromatic amines of the formula

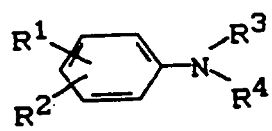

,

wherein

$R^1$ and $R^2$ independently of one another can denote hydrogen or straight-chain or branched $C_1$-$C_5$-alkyl or $C_1$-$C_5$-alkoxy, fluorine, chlorine, bromine or cyano or a fused benzene ring which can be substituted by methyl, ethyl, methoxy or ethoxy, and

$R^3$ and $R^4$ independently of one another represent straight-chain or branched $C_1$-$C_{10}$-alkyl or $C_3$-$C_{10}$-cycloalkyl, it being additionally possible for $R^4$ to represent hydrogen,

by reacting aromatic amines of the formula

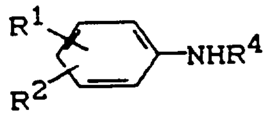

,

with alkanols of the formula

$R^3OH$

or the corresponding ethers of the formula

$R^3$-O-$R^3$,

$R^1$, $R^2$, $R^3$ and $R^4$ having the above meaning,
or by reacting aromatic amines of the formula

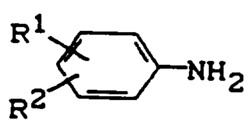

,

with aromatic amines of the formula

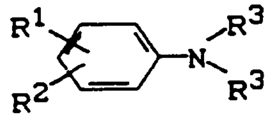

,

$R^1$, $R^2$ and $R^3$ having the above meaning,
in the gas phase or liquid phase at an elevated temperature, according to Claim 1, characterized in that the reaction is carried out in the presence of niobic acid or tantalic acid at 160-400 °C, preferably at 200-330 °C.

3. Process according to Claim 1, characterized in that the niobic acid or tantalic acid is mixed in a quantitative ratio of from 5:95 to 99:1 with an inert solid such as titanium dioxide, zinc oxide, magnesium oxide, iron oxide, silicon dioxide, graphite or α-alumina, and the mixture is pelletised.

4. Process according to Claim 2, characterized in that 0.3-20 mol of alkanol per mol of aromatic amine are employed in the reaction.

5. Process according to Claim 2, characterized in that, in the case of reaction in the gas phase, a catalyst loading of 0.1-4 l/l/h, preferably 0.3-2 l/l/h, of aromatic amine to be alkylated per litre of catalyst per hour is set.

6. Process according to Claim 2, characterized in that, in the case of reaction in the liquid phase, niobic acid or tantalic acid is used in a quantity of 2-25 % by weight, preferably 4-20 % by weight, relative to the aromatic amine to be alkylated.

7. Process according to Claim 2, characterized in that the reaction in the gas phase is preferably carried out at 200-330 °C.

8. Process according to Claim 2, characterized in that the reaction is carried out at 160-220 °C for preferential N-monoalkylation as against N,N-dialkylation and at 200-330 °C for preferential N,N-dialkylation as against N-monoalkylation.

9. Process for the preparation of aromatic amines dialkylated, preferably diethylated, on the N-atom, according to Claim 1, characterized in that the reaction is carried out at 200-330 °C, preferably at 220-310 °C, and with a molar ratio of aniline to alkanol or the corresponding dialkyl ether of 1:2-10 or 1:1-5 respectively, preferably 1:3-8 or 1:1.5-4 respectively, and particularly preferably 1:3-6 or 1:1.5-3 respectively.

10. Process according to Claim 1, characterized in that the catalyst has a BET surface area of 5-350 $m^2/g$, preferably 50-350 $m^2/g$, particularly preferably 100-250 $m^2/g$ and very particularly preferably 100-200 $m^2/g$.

**Revendications**

1. Procédé de préparation d'amines aromatiques alkylées sur l'atome d'azote à partir d'amines aromatiques et d'alcools inférieurs, caractérisé en ce que l'on effectue la réaction en présence d'acide niobique ou d'acide tantalique.

2. Procédé de préparation d'amines aromatiques de formule

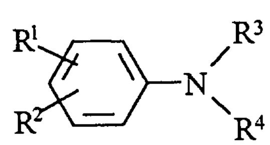

,

dans laquelle

$R^1$ et $R^2$ peuvent représenter indépendamment l'un de l'autre un atome d'hydrogène ou un reste alkyle en $C_1$-$C_5$ ou alcoxy en $C_1$-$C_5$ linéaire ou ramifié, fluoro, chloro, bromo ou cyano, ou un noyau benzène condensé qui peut être substitué par méthyle, éthyle, méthoxy ou éthoxy, et

$R^3$ et $R^4$ représentent indépendamment l'un de l'autre un reste alkyle en $C_1$-$C_{10}$ linéaire ou ramifié ou cycloalkyle en $C_3$-$C_{10}$, $R^4$ pouvant en outre représenter un atome d'hydrogène,

par réaction d'amines aromatiques de formule

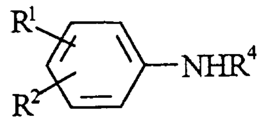

,

avec des alcanols de formule

$R^3OH$

ou les éthers correspondants de formule

$R^3$-O-$R^3$,

$R^1$, $R^2$, $R^3$ et $R^4$ ayant la signification donnée ci-dessus,
ou par réaction d'amines aromatiques de formule

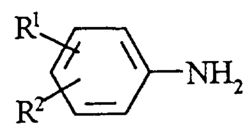

,

avec des amines aromatiques de formule

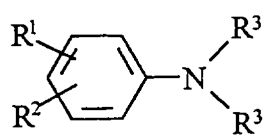

,

$R^1$, $R^2$ et $R^3$ ayant la signification donnée ci-dessus,

en phase gazeuse ou en phase pâteuse à température élevée selon la revendication 1, caractérisé en ce que l'on effectue la réaction en présence d'acide niobique ou d'acide tantalique à 160-400 °C, de préférence à 200-330 °C.

3. Procédé selon la revendication 1, caractérisé en ce que l'on mélange et on transforme en boulettes l'acide niobique ou l'acide tantalique avec une substance solide inerte comme le dioxyde de titane, l'oxyde de zinc, l'oxyde de magnésium, l'oxyde de fer, le dioxyde de silicium, le graphite ou l'oxyde d'aluminium $\alpha$, en des proportions de 5:95 à 99:1.

4. Procédé selon la revendication 2, caractérisé en ce que l'on utilise dans la réaction 0,3 - 20 moles d'alcanol par mole d'amine aromatique.

5. Procédé selon la revendication 2, caractérisé en ce que, dans la réaction en phase gazeuse, on établit une charge de catalyseur de 0,1 - 4 l/l/h, de préférence de 0,3 - 2 l/l/h, d'amine aromatique à alkyler par litre de catalyseur par heure.

6. Procédé selon la revendication 2, caractérisé en ce que, dans la réaction en phase pâteuse, on utilise l'acide niobique ou l'acide tantalique en une quantité de 2 - 25 % en masse, de préférence de 4 - 20 % en masse, par rapport à l'amine aromatique à alkyler.

7. Procédé selon la revendication 2, caractérisé en ce que, en phase gazeuse, on travaille de préférence à 200-330 °C.

**8.** Procédé selon la revendication 2, caractérisé en ce que, pour favoriser la N-monoalkylation par rapport à la N,N-dialkylation, on travaille à 160-220 °C, et pour favoriser la N,N-dialkylation par rapport à la N-monoalkylation, on travaille à 200-330 °C.

**9.** Procédé de préparation d'amines aromatiques dialkylées, de préférence diéthylées, sur l'atome d'azote, selon la revendication 1, caractérisé en ce que l'on travaille à 200-330 °C, de préférence à 220-310 °C, et avec un rapport molaire respectifs de l'aniline à, respectivement, l'alcanol ou l'éther dialkylique correspondant, de 1:2-10 ou 1:1-5, de préférence de 1:3-8 ou 1:1,5-4, de façon particulièrement préférée de 1:3-6 ou 1:1,5-3.

**10.** Procédé selon la revendication 1, caractérisé en ce que le catalyseur a une surface BET de 5-350 $m^2/g$, de préférence de 50-350 $m^2/g$, de façon particulièrement préférée de 100-250 $m^2/g$, de façon tout particulièrement préférée de 100-200 $m^2/g$.